Europäisches Patentamt

European Patent Office

Office européen des brevets

(19)

(11) Veröffentlichungsnummer: **0 314 951**
**B1**

(12) # EUROPÄISCHE PATENTSCHRIFT

(45) Veröffentlichungstag der Patentschrift:
**27.12.90**

(51) Int. Cl.⁵: **A61F 2/34**

(21) Anmeldenummer: **88116955.1**

(22) Anmeldetag: **12.10.88**

(54) Hüftgelenk-Pfannenprothese.

(30) Priorität: **03.11.87 DE 8714635 U**

(43) Veröffentlichungstag der Anmeldung:
**10.05.89 Patentblatt 89/19**

(45) Bekanntmachung des Hinweises auf die Patenterteilung:
**27.12.90 Patentblatt 90/52**

(84) Benannte Vertragsstaaten:
**AT DE ES FR GB GR IT NL SE**

(56) Entgegenhaltungen:
**EP-A- 0 120 595**
**DE-A- 3 205 526**
**DE-U- 8 714 635**
**FR-A- 2 519 545**
**GB-A- 2 080 118**

(73) Patentinhaber: **Waldemar Link GmbH & Co,**
**Barkhausenweg 10, D-2000 Hamburg 63(DE)**

(72) Erfinder: **Keller, Arnold, An der Naherfurth 5,**
**D-2061 Kayhude(DE)**

(74) Vertreter: **Glawe, Delfs, Moll & Partner Patentanwälte,**
**Postfach 26 01 62 Liebherrstrasse 20,**
**D-8000 München 26(DE)**

**Beschreibung**

Die Erfindung betrifft eine Hüftgelenk-Pfannenprothese mit Einrichtungen zur Verankerung am Knochen, mit einer Fassung und mit einem die Gelenkfläche bildenden Einsatz, wobei die Fassung mindestens eine nach außen vorragende Stütze zur Überbrückung eines Zwischenraums zwischen der Fassung und der Oberfläche des Knochens aufweist und wobei die Stütze durch an ihr und der Fassung vorgesehene, zusammenwirkende Halterungseinrichtungen in der Fassung gehalten ist.

Die bekannten Einrichtungen zur zementlosen Verankerung von Pfannenprothesen im Knochen, wie beispielsweise Gewindegänge an der Außenseite der Fassung einer Metallschale (DE-A 2 950 536) setzen voraus, daß sie unmittelbar in das die Prothese im wesentlichen über den vollen Umfang abstandsfrei umgebende Knochengewebe eingreifen können. Wenn im Falle einer Reoperation die Acetabulum-Öffnung durch Lageveränderung der zu ersetzenden Pfanne nach cranial derart ausgeweitet ist, daß sich die Pfanneneingangsebene nicht mehr anatomisch kreisrund, sondern oval darstellt und sich die ursprüngliche konkave Kugelflächenform des Acetabulums derart verändert hat, daß sich unterhalb der ovalär veränderten Pfanneneingangsebene eine kavernenförmige Ausbildung entwickelt hat, ist eine erneute stabile Versorgung mit einer Acetabulumpfanne in jedem Fall problematisch. Ein weiteres Ausbohren des Acetabulums bis zur Wiedererlangung einer kreisrunden, halbkugelig konkaven Form, müßte sich an dem Maß der größten ovalen Ausdehnung orientieren und darüber hinaus auch die kavernenförmige Ausbildung erfassen. Dies ist in Richtung quer zur Richtung der größten Ausdehnung der Pfanneneingangsebene unter Einbezug der kavernenförmigen Ausbildung wegen Fehlens der dafür notwendigen Knochensubstanz in dieser Richtung meistens nicht möglich. Insbesondere nach ventral ergibt eine normale Beckenschaufel dafür nicht die notwendige Ausdehnung, so daß ein derartiges Vorgehen über den ventral zur Verfügung stehenden Bereich hinausgeht und den Zusammenhalt der Beckenschaufel von caudal nach cranial öffnet und damit instabil macht. Als weiterer Nachteil eines derartigen Vorgehens ist der übergroße weitere Knochenverlust anzusehen. Die Beckenschaufel würde dadurch noch mehr an Stabilität verlieren und gleichfalls würden dadurch Maßnahmen, die in der weiteren Zukunft evtl. notwendig werden, erheblich eingeschränkt.

In derartigen Fällen behilft man sich vielfach mit der Ausfüllung des ovalär geformten Pfannendefektes einschließlich der Kaverne durch Knochenzement und darin die erneute Einbettung einer künstlichen Hüftpfanne. Der eingebrachte Knochenzement verhindert bei einem derartigen Vorgehen nicht nur eine knöcherne Rekonstruktion, sondern führt erwiesenermaßen eher zur weiteren Destruktion. Gleichfalls ist die erreichbare Stabilität als sehr limitiert anzusehen. Als weitere Maßnahme ist das Anbringen von Metallimplataten bekannt, deren konkave Halbschale zur Aufnahme der künstlichen Hüftpfanne, in den Defektbereich des Acetabulums hineinreichen, und an deren Rändern nach cranial und caudal Laschen aus Metall angebracht sind und mittels Knochenschrauben an der Beckenschaufel fixiert werdewn. Problematisch ist dabei ebenfalls die Stabilität der möglichen Fixierung. Gelegentlich wird ebenfalls versucht, den ovalen Defektbereich einschließlich der Kaverne mit autologem bzw. homologem Knochenmaterial auszufüllen, um da hinein eine erneute Fixierung der künstlichen Hüftpfanne mit gleichzeitig knöcherner Rekonstruktion zu erreichen.

Verständlicherweise ist bei dieser Maßnahme die erreichbare primäre Stabilität in hohem Maße unsicher und der Erfolg bleibt selbst bei längerfristigem Ruhigstellen des Patienten zweifelhaft.

Es sind Hüftgelenk-Pfannenprothesen bekannt (EP-A 0 120 595, GB-A 2 080 118), die mit Knochenzement in einer regelmäßig sphärisch gefrästen Knochenhöhlung zu verankern sind. Um eine bestimmte Zementdicke zwischen der Außenfläche der Prothese und der Knochenoberfläche zu garantieren, ist die Fassung dieser Hüftpfannen mit äußeren Vorsprüngen entsprechender Länge versehen. Da der Knochenzement die Verbindung zwischen Prothese und Knochen übernimmt, haben diese Vorsprünge keine selbständige Stützfunktion. Da sie eine bestimmte, unveränderliche Länge aufweisen und für den Operateur auch nicht austauschbar sind, sind sie nicht geeignet zur individuellen Abstützung in unregelmäßig geformten Bereichen des Knochen. Das gilt auch für diejenige bekannte Prothese (EP-A 0 120 595), bei welcher die Vorsprünge nicht einstückig mit der Fassung verbunden sind, sondern von der Innenseite in Bohrungen der Fassung einsetzbar sind und darin durch den Einsatz gehalten werden, der fabrikmäßig unveränderbar mit der Fassung verbunden wird.

Der Erfindung liegt die Aufgabe zugrunde, eine Verankerungseinrichtung für eine Hüftgelenk-Pfannenprothese zu schaffen, die auch in diesen Fällen hinreichende Verankerung verspricht und eine knöcherne Rekonstruktion nicht ausschließt.

Die erfindungsgemäße Lösung besteht in den kennzeichnenden Merkmalen des Anspruchs 1.

Die Stütze ist so ausgebildet, daß sie nach dem Einsetzen der Pfanne von dieser aus vorgestreckt werden kann, und zwar durch Betätigung von der Pfanneninnenseite her, da die Außenseite nicht mehr zugänglich ist. Zu diesem Zweck sind die Halterungseinrichtungen, die die Stütze mit der Pfanne verbinden, so ausgebildet, daß sie die nachträgliche Lageänderung der Stütze und die Übertragung der Verankerungskräfte ermöglichen. Neben anderen Bauarten, beispielsweise einer Bajonetthalterung, ist dazu nach der Erfindung vornehmlich die Ausbildung der prothesenseitigen Halterung als Gewindebohrung geeignet, wobei die Stütze als Stift mit zumindest an seinem pfannenseitigen Ende vorgesehenen entsprechenden Gewinde ausgebildet ist. Die Stütze mag schon beim Einsetzen der Pfanne in der Halterung vorhanden sein; zweckmäßiger ist es, eine Vielzahl von Halterungen bzw. Gewindebohrungen in der Pfanne vorzusehen, damit nach dem Einsetzen der Pfanne diejenige Halterung ausgewählt werden kann, die die günstigste Lage im Hinblick auf die gewünschte Stützenstellung aufweist. Eine Vielzahl solcher Halterungen ist auch deshalb zweckmäßig, weil im allgemeinen eine Stütze nicht ausreicht, sondern deren mehrere verwendet werden müssen.

Die Ausbildung der Stütze als ein von der Innenseite der Pfanne durch die Halterungsöffnung durchzusteckender Stift beschränkt deren Querschnittsgröße und damit die Größe der an der Knochenoberfläche zur Verfügung stehenden Stützfläche. Wenn eine größere Stützfläche gewünscht ist, kann daher das knochenseitige Ende der Stütze mit einem Stützteller versehen sein, der schon vor der Implantation der Pfanne an geeigneter Stelle der Knochenoberfläche fixiert werden kann oder an der Außenseite der Pfanne im Bereich der zu verwendenden Halterung befestigt ist derart, daß er sich beim Vorschieben der Stütze mit dieser verbindet.

Im Hinblick auf eine möglichst große Abstützfläche der Stütze kann diese am knochenseitigen Ende massiv ausgeführt sein. Jedoch kann es zweckmäßig sein, eine hohle Stütze zu verwenden, um eine Knochenschraube hindurchführen zu können.

Nach dem Einsetzen der Pfanne stellt der Operateur bei jeder zu verwendenden Halterung mittels einer Tiefenlehre den Abstand zur Knochenoberfläche und damit die Länge der auszuwählenden Stütze fest. Will man dies vermeiden, so kann man nach der Erfindung Stützen verwenden, die aus mehreren teleskopisch ineinander verschraubbaren Hülsen bestehen. Diese können nach einem weiteren Merkmal im zurückgezogenen Zustand eine gemeinsame Länge haben, die nicht wesentlich größer ist als die Dicke des die Gewindebohrung enthaltenden Pfannen teils, so daß sie schon beim Einsetzen der Pfanne darin enthalten sein können und/oder für jeden Abstand zwischen Pfanne und Knochen verwendbar sind.

Die Erfindung wird im folgenden näher unter Bezugnahme auf die Zeichnung erläutert, die vorteilhafte Ausführungsbeispiele veranschaulicht. Darin zeigen:

Fig. 1 eine Darstellung der implantierten Prothese und des umgebenden Knochens im Sagittalschnitt,
Fig. 2 eine Außenansicht in Richtung der Pfannenachse,
Fig. 3 einen Axialschnitt durch die Pfanne,
Fig. 4 und 5 durch zwei hohl bzw. massiv ausgebildete Stützen,
Fig. 6 und 7 Seitenansicht von Stützen gemäß Fig. 4 mit unterschiedlicher Länge,
Fig. 8 und 9 eine Drauf- und Schnittansicht eines Stütztellers und
Fig. 10 und 11 eine Teleskopstütze sowie eine Schnittansicht der sie bildenden Teleskophülsen.

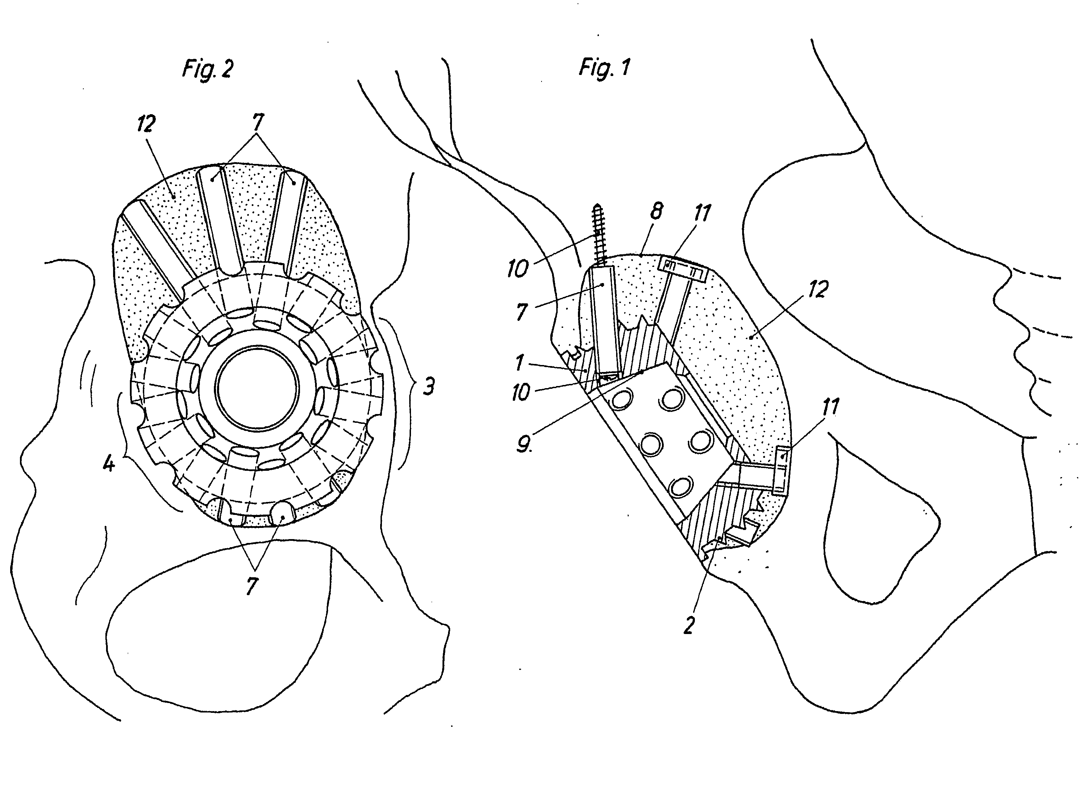

Gemäß Fig. 1 und 2 ist das Acetabulum durch Wanderung einer reoperierten Pfannenprothese in dem punktiert dargestellten Bereich des Hüftbeins nach cranial derart stark oval erweitert, daß der dargestellte Prothesenteil 1, der die metallene Fassung einer Pfannenprothese bildet, die durch einen nicht dargetellten Kunststoffeinsatz zu ergänzen ist, mit ihren am Umfang vorgesehenen, als Gewinde ausgebildeten Verankerungsvorsprüngen 2 das Knochengewebe nur seitlich in den Zonen 3 und 4 unmittelbar erreicht, was bei zementfreier Verankerung unzureichend ist.

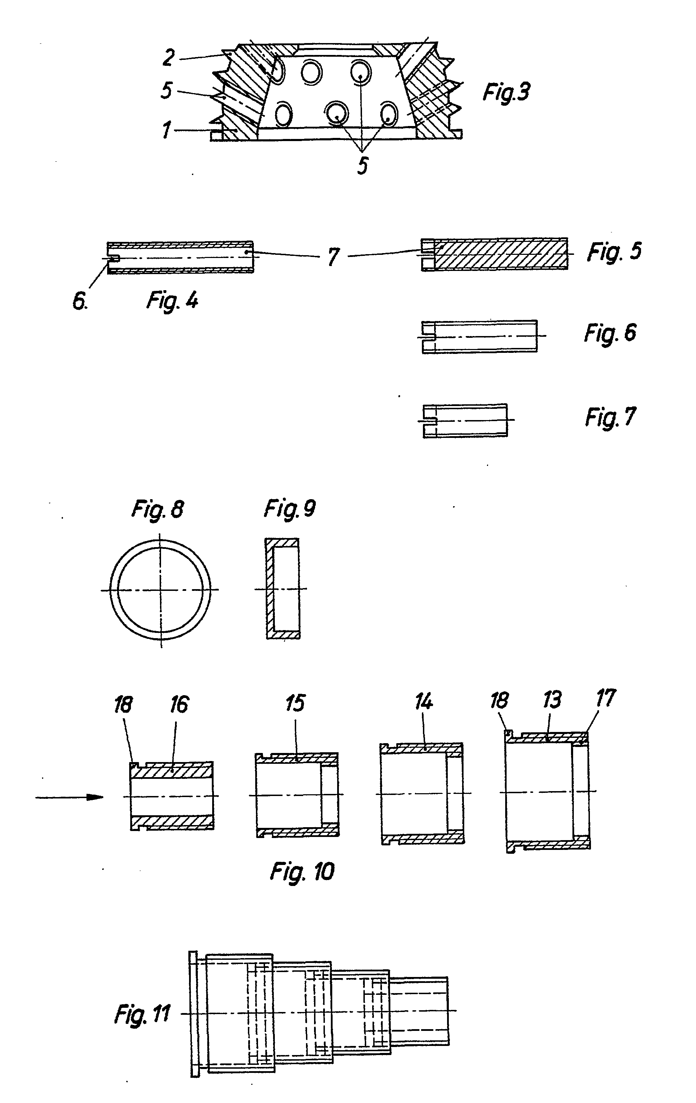

Wie man vor allem in Fig. 2 und 3 erkennt, ist die Fassung mit einer Vielzahl von Gewindebohrungen 5 versehen, die gleichmäßig in mehreren Reihen über den Umfang verteilt sind. Die Bohrungen liegen in gemeinsamen Ebenen mit der Pfannenachse und sind in einem solchen Winkel angeordnet, daß die Verlängerung ihrer Mittelachsen ungefähr zum Gelenkmittelpunkt weist. Der mit der Pfannenachse eingeschlossene Winkel beträgt bei der öffnungsnäheren Reihe etwa 50 bis 90° und bei der öffnungsferneren Reihe etwa 35 bis 50°. Jeweils etwa 6 bis 10, nämlich 8 Löcher sind in jeder Reihe über den Umfang verteilt. Insgesamt liegen die Stützenwinkel zweckmäßigerweise zwischen 35 und 90° zur Pfannenachse.

Als Stützen sind Gewindestifte vorgesehen, die gemäß Fig. 4 bis 7 hohl oder massiv sein können, unterschiedliche Länge aufweisen und an einem Ende Schlitze 6, einen Innensechskant oder dergleichen zum Angriff eines Drehwerkzeugs enthalten. Gemäß Fig. 2 sind sowohl oberhalb als auch unterhalb der Prothese je drei derartige Stützen 7 verwendet, deren Länge so ausgewählt ist, daß sie einerseits bis zur Knochenoberfläche 8 vorragen und ggf. darin auch ein wenig eingedrückt sind und andererseits nach innen nicht über die zur Aufnahme des Einsatzes vorgesehene Fläche 9 vorragen.

Während die in Fig. 2 dargestellten Stützen ohne weitere Hilfsmittel an der Knochenoberfläche anliegen und dadurch fixiert sind, ist bei einer der in Fig. 1 erscheinenden Stütze eine Knochenschraube 10 vorgesehen, die die hohl ausgebildete Stütze durchdringt. Bei zwei anderen Stützen sind Stützteller 11 zur Verminderung der am Knochen wirksamen Flächenpressung vorgesehen, die von der in Fig. 8 und 9 dargestellten Art sein können und lediglich durch Einklemmung zwischen der Knochenoberfläche 8 und den Stützen 7 gehalten sind. Statt dessen können auch besondere Verbindungsorgane zur festen Verbindung mit den Stützen vorgesehen sein.

Der Hohlraum 12 zwischen Prothese und Knochenoberfläche 8 wird mit Knochenmaterial ausgefüttert, um eine Rekonstruktion des Knochengewebes in diesem Bereich zu fördern, das sich je nach Schnelligkeit des Wachstums nach einigen Wochen bis Monaten an der Kraftübertragung auf die Prothese beteiligt. Bis dahin gewährleisten die Stützen eine primäre Funktions- und Übungsstabilität.

Die gemäß Fig. 10 und 11 teleskopisch aufgebaute Stütze besteht aus mehreren Teleskophülsen 13, 14, 15 16, von denen die größte 13 ein den Gewindebohrungen der Pfanne entsprechendes Außengewinde und ein dem Außengewinde der nächst kleineren Teleskophülse 14 entsprechendes Innengewinde besitzt, usw. bis zur kleinsten Hülse 16, die kein Innengewinde aber ggf. eine Bohrung zur Aufnahme einer Knochenschraube sowie einen Innensechskant für ein Drehwerkzeug enthält. Die drei größeren Hülsen weisen innen an demjenigen Ende, das später nach außen weisen soll, einen gewindelosen Bund 17 auf, während am anderen Ende ein äußerer gewindeloser Bund 18 vorgesehen ist, der mit dem Bund 17 derart zusammenwirkt, daß keine Hülse aus der nächst größeren nach außen herausgeschraubt werden kann.

Im Ausgangsstadium liegen alle Hülsen ineinander, so daß die Gesamtlänge der Länge jeder der dargestellten Hülsen entspricht und nicht oder nicht wesentlich größer ist als die Länge einer sie aufnehmenden Bohrung 5 der Prothese. Wird nun mittels eines geeigneten Werkzeugs die Hülse 16 gedreht, so verlagern sich die Hülsen sukzessive in Pfeilrichtung (Fig.10) nach außen, bis die Hülse 16 die Knochenoberfläche 8 erreicht hat.

Sowohl für die Gewindebolzen 7 als auch für die Teleskopstützen 13 bis 16 können geeignete Einrichtungen zur Fixierung der erreichten Rotationsstellung vorgesehen sein, die dem Stand der Technik entnommmen werden können.

Nach der Verankerung des Prothesenteils 1 kann der die Gelenkfläche bildende Einsatz eingefügt werden.

Der die Halterungen 5 für die Stützen bildende Prothesenteil kann von beliebiger Art sein. Man erkennt aber an den Ausführungsbeispielen, daß die Erfindung sich mit besonderem Vorteil bei solchen Pfannenprothesen anwenden läßt, die aus einem Fassungsteil 1 aus starrem Material, der dann die Halterungen enthält, und einem besonderen Einsatz bestehen.

Die Stützen sind in den Beispielen zur Überbrückung eines Abstands zwischen Prothesenoberfläche und Knochenoberfläche eingesetzt. Es kann Fälle geben, in denen man sich ihrer auch dann bedient, wenn ein solcher Abstand nicht besteht, nämlich lediglich zum Zwecke besserer Verankerung im Knochen, wobei die verwendete Stütze, die am Ende zugespitzt sein kann, ein wenig in die Knochenoberfläche eindringt, ohne jedoch in der Art einer Knochenschraube Zukräfte zu übertragen.

**Patentansprüche**

1. Hüftgelenk-Pfannenprothese, mit Einrichtungen (2) zur Verankerung an einem die Prothese aufnehmenden Knochen, mit einer Fassung (1) und mit einem die Gelenkfläche bildenden Einsatz, wobei die Fassung mindestens eine nach außen vorragende Stütze (7, 13–16) zur Überbrückung eines Zwischenraums zwischen der Fassung und der Oberfläche (8) des Knochens aufweist und wobei die Stütze durch an ihr und der Fassung vorgesehene, zusammenwirkende Halterungseinrichtungen in der Fassung gehalten ist, dadurch gekennzeichnet, daß die Halterungseinrichtungen derart ausgebildet sind, daß die Stütze (7, 13–16) nach dem Einsetzen der Fassung von der Fassungsinnenseite her in eine Richtung ihrer Längsachse nach außen vorstreckbar und in beiden Richtungen ihrer Längsachse arretierbar ist, und daß der Einsatz nach der Verankerung der Fassung in diese einsetzbar ist.

2. Prothese nach Anspruch 1, dadurch gekennzeichnet, daß die Halterungseinrichtung in der Fassung von Gewindebohrungen (5) gebildet sind und die Stütze (7, 13–16) als Stift mit zumindest an seinem pfannenseitigen Ende vorgesehenem Gewinde ausgebildet ist.

3. Prothese nach Anspruch 2, dadurch gekennzeichnet, daß eine Vielzahl von Gewindebohrungen (5) vorgesehen ist.

4. Prothese nach einem der Ansprüche 1 bis 3, dadurch gekennzeichnet, daß die Stütze (7, 13–16) am knochenseitigen Ende mit einem Stützteller (11) versehen ist.

5. Prothese nach einem der Ansprüche 1 bis 4, dadurch gekennzeichnet, daß die Stütze (7, 13–16) hohl ist zur Aufnahme einer Knochenschraube (10).

6. Prothese nach einem der Ansprüche 1 bis 5, dadurch gekennzeichnet, daß die Stütze mehrere teleskopisch ineinander verschraubbare Hülsen (13–16) umfaßt.

7. Prothese nach Anspruch 6, dadurch gekennzeichnet, daß die Teleskophülsen (13–16) im zurückgezogenen Zustand eine gemeinsame Länge haben, die nicht wesentlich größer als die Dicke des die Gewindebohrung (5) enthaltenden Pfannenteils (1) ist.

**Claims**

1. A hip-joint acetabular prosthesis, with means (2) for anchoring to a bone accommodating the prosthesis, with a socket member (1) and with an insert forming the joint surface, wherein the socket member has at least one outwardly projecting support (7, 13–16) for spanning a clearance between the socket member and the surface (8) of the bone, and wherein the support is retained in the socket member by co-operating retaining means provided thereon and on the socket member, characterised in that the retaining means are so formed that, after the insertion of the socket member, the support (7, 13–16) can be extended outwards from the inside of the socket member in one direction of its longitudinal axis and can be locked in both directions of its longitudinal axis, and in that after the anchoring of the socket member the insert can be inserted therein.

2. A prosthesis according to Claim 1, characterised in that the retaining means in the socket member are formed by threaded bores (5) and the support (7, 13–16) is in the form of a pin provided with a thread at least at its end adjacent the acetabulum.

3. A prosthesis according to Claim 2, characterised in that a plurality of threaded bores (5) are provided.

4. A prosthesis according to any one of Claims 1 to 3, characterised in that at its end adjacent the bone the support (7, 13–16) is provided with a support plate (11).

5. A prosthesis according to any one of Claims 1 to 4, characterised in that the support (7, 13–16) is hollow so as to accommodate a bone screw (10).

6. A prosthesis according to any one of Claims 1 to 5, characterised in that the support comprises a plurality of sleeves (13–16) which can be screwed telescopically into one another.

7. A prosthesis according to Claim 6, characterised in that in their retracted condition the telescopic sleeves (13–16) are of a common length which is not substantially greater than the thickness of the acetabular part (1) including the threaded bore (5).

## Revendications

1. Prothèse cotyloïdienne, comportant des dispositifs (2) destinés à un ancrage à un os recevant la prothèse, une monture (1) et une pièce d'insertion formant la surface de l'articulation, la monture possédant au moins un support (7, 13–16), en saillie vers l'extérieur, destiné à recouvrir un espace intermédiaire situé entre la monture et la surface (8) de l'os, le support étant maintenu dans la monture par des dispositifs d'attache, concourants, prévus contre lui et contre la monture, caractérisée en ce que les dispositifs d'attache sont constitués de telle sorte que le support (7, 13–16) peut, après mise en place de la monture, être étiré vers l'extérieur, à partir du côté intérieur de la monture, dans l'un des sens de son axe longitudinal, et peut être bloqué dans les deux directions de son axe longitudinal, et que la pièce d'insertion, après ancrage de la monture, peut être insérée dans cette dernière.

2. Prothèse selon la revendication 1, caractérisée en ce que les dispositifs d'attache aménagés dans la monture sont constitués de trous taraudés (5), et que le support (7, 13–16) est conçu comme une tige, comportant un filetage, prévue au moins en son extrémité située du côté glénoïde.

3. Prothèse selon la revendication 2, caractérisée en ce qu'on prévoit un grand nombre de trous taraudés (5).

4. Prothèse selon l'une des revendications 1 à 3, caractérisée en ce que le support (7, 13–16) est prévu, à l'extrémité côté os, avec un disque d'appui (11).

5. Prothèse selon l'une des revendications 1 à 4, caractérisée en ce que le support (7, 13–16) est creux, pour recevoir une vis à os (10).

6. Prothèse selon l'une des revendications 1 à 5, caractérisée en ce que le support comporte plusieurs douilles (13–16), pouvant être vissées les unes dans les autres d'une manière télescopique.

7. Prothèse selon la revendication 6, caractérisée en ce que les douilles télescopiques (13-16) ont, à l'état rétracté, une longueur commune, qui n'est pas très supérieure à l'épaisseur de la partie glénoïde (1) contenant le trou taraudé (5).

Fig. 2

Fig. 1

EP 0 314 951 B1

EP 0 314 951 B1

Fig. 3

Fig. 4

Fig. 5

Fig. 6

Fig. 7

Fig. 8

Fig. 9

Fig. 10

Fig. 11